# EUROPEAN PATENT SPECIFICATION

(11) **EP 1 967 171 B1**
(45) Date of publication and mention of the grant of the patent: **13.06.2012**
(21) Application number: 06745658.2
(22) Date of filing: 25.04.2006
(51) Int. Cl.: A61F 13/02, A61K 9/70

(54) **Production method of an adhesive patch**
Methode zur Erstellung von einem Pflaster
Méthode de production d'un patch adhésif

(30) Priority: 28.12.2005 JP 2005377554
(43) Date of publication of application: 10.09.2008
(73) Proprietor: TEIKOKU SEIYAKU CO., LTD., Higashikagawa-shi, Kagawa 769-2695 (JP)
(72) Inventor: UEMATSU, Masanori, Kagawa 7612300 (JP)
(74) Representative: Albrecht, Thomas
(86) International application number: PCT/JP2006/308630
(87) International publication number: WO 2007/077640

(56) References cited:
- EP-A1- 0 360 458
- EP-A1- 0 630 629
- EP-A2- 0 434 258
- FR-A1- 2 794 969
- JP-A- 2000 351 727
- JP-A- 2004 188 005
- JP-A- 2004 215 743
- JP-U- 04 120 727
- US-A1- 2005 232 982

## Description

### TECHNICAL FIELD

The present invention relates to a production method of adhesive patches, such as poultices, for the treatment of inflammation, pain, itch and other symptoms. More particularly, the present invention relates to a production method of an efficient adhesive patch that can be easily applied not only by non-disabled people, but also by aged people and patients with decreased grip strength, by facilitating the sequence of actions required to apply the patch, from the removal of the liner (liner) to the application of the patch. The adhesive patch produced by the method of the present invention can be applied in a safe and hygienic manner since the drug-containing matrix does not come into contact with hands during the application.

### BACKGROUND ART

Anti-inflammatory and analgesic poultices are now widely used in the conservative treatment of lumbago, joint pain, shoulder stiffness and other symptoms caused by aging or hard working. Adhesive patches are also widely used in the treatment of herpes zoster in recent years.

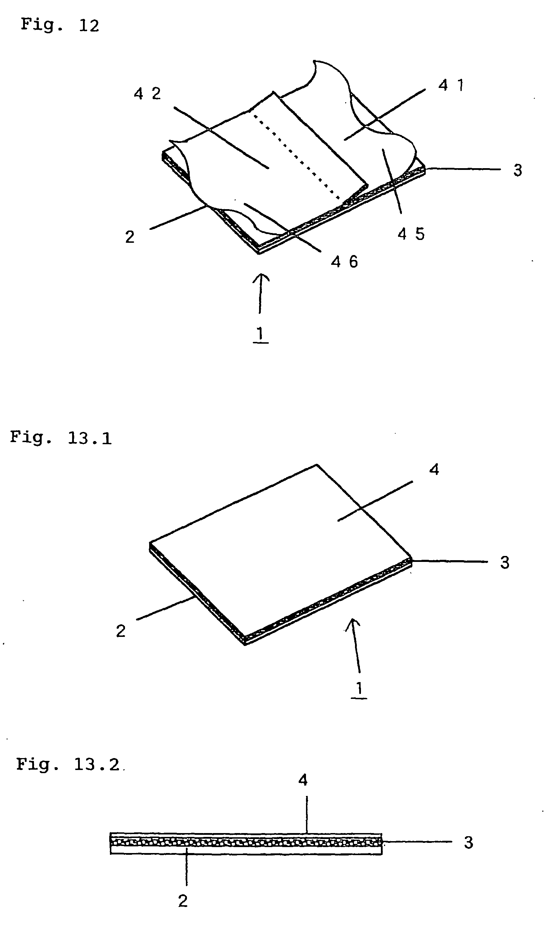

Conventional adhesive patches, such as poultice-type patches, typically have a multi-layer structure. As an example, a conventional adhesive patch 1 is shown in Fig. 13. The adhesive patch 1 has a multi-layer structure including a white or skin-colored backing 2, an adhesive drug-containing matrix 3 that is spread substantially entirely over one surface of the backing, and a sheet of liner 4 that entirely covers the drug-containing matrix.

Typically, such an adhesive patch (such as a poultice-type patch) is applied in the following manner: First, the user rubs the edge of the poultice-type patch with a substantial force to cause the liner 4 to slide relative to the surface of the drug-containing matrix 3. This causes the film to partially come off the surface of the matrix. The user quickly holds the drug-containing matrix with one finger before the film sticks again to the matrix and, using another finger, carefully removes the liner 4 so that the adhesive matrix will not stick to itself. Once the liner 4 is completely peeled, the user, holding the drug-containing matrix and the backing, applies the adhesive patch to the application site.

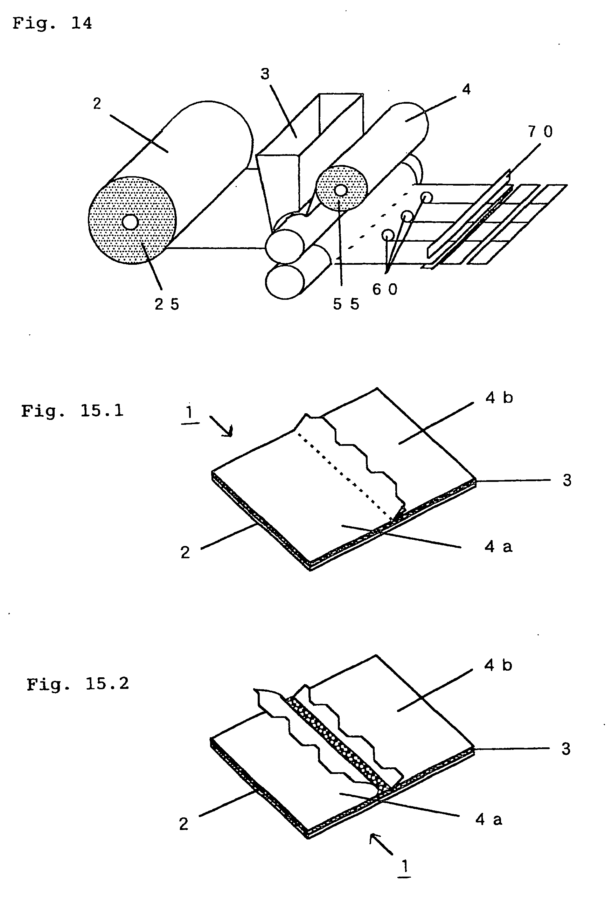

This type of adhesive patch is essentially produced in the following method: As shown in Fig. 14, the adhesive drug-containing matrix 3 is spread substantially entirely over one surface of a backing 2 as the backing 2 is drawn from a backing roll 25. The liner 4, drawn from a liner roll 55, then adheres to and is laminated onto the surface of the drug-containing matrix to form a laminate, which in turn is cut into strips 60, and then into pieces with a predetermined size 70.

A problem with these adhesive patches in which a single sheet of liner is used to cover the entire surface of the drug-containing matrix is that users often have difficulty applying these patches to the application site since the liner of these patches is difficult to remove from the surface of the matrix, often resulting in the matrix sticking to itself.

One proposed solution to this problem is an adhesive patch shown in Fig. 15 (Patent Document 1). The adhesive patch 1 includes a white or skin-colored backing 2, an adhesive drug-containing matrix 3 that is spread substantially entirely over one surface of the backing, and a pair of liners, an upper liner 4a and a lower liner 4b, that adheres to the drug-containing matrix.

To apply this adhesive patch, the user first pinches the tab of the upper liner 4a with his fingers and carefully removes the film 4a. The user then pinches the tab of the lower liner 4b with his fingers and carefully removes the film 4b so that the adhesive drug-containing matrix will not stick to itself. Once the film is completely peeled, the user, holding the drug-containing matrix and the backing, applies the patch to the application site.

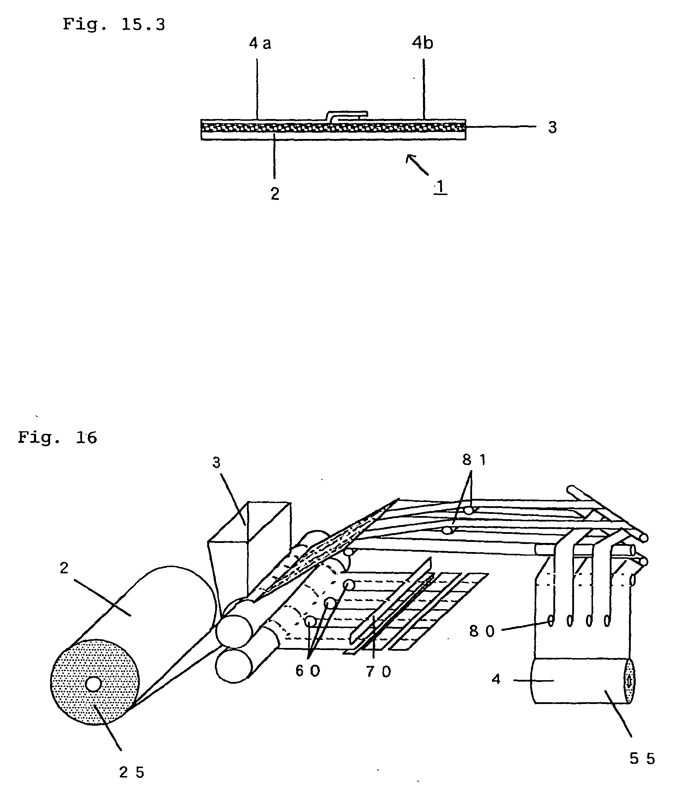

This type of adhesive patch can be produced in the following method: As shown in Fig. 16, the adhesive drug-containing matrix 3 is spread substantially entirely over one surface of a backing 2 as the backing 2 is drawn from the backing roll. The liner 4, drawn from the liner roll 55, then adheres to and is laminated onto the surface of the drug-containing matrix. Upon this, the liner 4 is cut 80 into an upper liner 4a and a lower liner 4b, which are overlapped at their ends by a tab folder 81 and laminated onto the drug-containing matrix 3 to form a laminate. The laminate is then cut in half into strips 60, and then into pieces with a predetermined size 70.
Patent Document 1 Japanese Laid-Open Patent Publication No. 2001-219622

The above-described conventional adhesive patches, such as poultice-type patches, have the following problems that have yet to be addressed:
(1) The adhesive patches, such as poultice-type patches, are fabricated by spreading a drug-containing matrix substantially entirely over one surface of a backing, and placing a liner over the drug-containing matrix to form a laminate, or an original sheet, which is then cut into a desired shape, such as rectangular. In these patches, the backing, the drug-containing matrix and the liner are laminated together with their outer edges lying along the same plane. The patches therefore have no part along the outer edge that the user can hold to start peeling the liner. Thus, in order to peel the liner, the user must rub the patch with his fingers along its outer edge. This requires a certain type of dexterity and cannot readily be accomplished by aged people or other people with decreased dexterity.

(2) The drug-containing matrix is made to be highly sticky so that it can firmly stick to the application site. Thus, peeling the liner from the patch requires a substantial force. The adhesive patch, such as poultice-type patch, designed to be pulled by the ends to break and remove the liner requires an even greater force, making the patch unsuitable for use by aged people.

(3) All of these adhesive patches, such as poultice-type patches, are designed to be applied to the application site either after the liner has been entirely removed or while the film is being removed. Thus, the drug-containing matrix may come into direct contact with the fingers, which often results in the drug-containing matrix sticking to itself or wrinkled. To avoid this and to ensure proper application of the patch, the user must carefully and slowly apply the patch to the application site.

(4) The drug-containing matrix that comes into direct contact with the fingers during the peeling of the liner loses the initial stickiness and, as a result, the once-applied adhesive patch such as poultice-type patch may partially come off the application site.

(5) The drug-containing matrix that comes into direct contact with the fingers is unfavorable from the hygienic point of view and requires much attention when applied to the damaged skin.

(6) The active ingredient and other components present in the drug-containing matrix may be transferred to the finger tips through the direct contact with the drug-containing matrix. The transferred drug-containing matrix is sticky and causes discomfort and must therefore be removed by rinsing.

(7) During the production method of the adhesive patches shown in Fig. 16, part of the lower liner 4b is folded to form the tab. To prevent this part from unfolding as it is folded in an in-line system, the method involves softening the material in the part by heating it, for example, by ultrasonic sealing. Also, the tab is folded in any desired size and formed by using nip rolls. Thus, materials with high melting points are not appropriately used in this method, which limits the types of materials that can be used in the method. Furthermore, the method tends to result in unstable product quality upon stopping and restarting of the machine operation and thus requires special attention, such as intensive checks and careful control.

US patent application US 2005/232982 A1 describes a patch containing fentanyl for mucous membrane of the oral cavity (oral transmucosal fentanyl), which rapidly increases the serum concentration of the drug, is easy in handling and is superior in safety. The patch can be prepared by laminating on one side of a drug layer which contains fentanyl or its salt as an active ingredient, methyl vinyl ether-maleic anhydride copolymer as an adhesive, and at least one substance selected from the group consisting of hydroxypropylcellulose, hydroxypropylmethylcellulose and hydroxyethylcellulose as a thickener, a support layer hardly soluble or insoluble in water, and a backing in their order.

European patent application EP 0 630 629 A1 describes a flexible wound dressing product including a thin-film layer, an adhesive layer, a backing layer which may be porous, an optional support layer, an optional release liner, and a hydrogel material. Where the backing layer is porous, the backing layer can be secured to the hydrogel material without the use of an adhesive. The wound dressing may also include a removable tab interposed between the thin film layer and release liner, providing a grippable surface for the removal of the release liner from the transparent thin film layer and to facilitate the handling of said wound dressing during application of the dressing to the wound.

French patent application FR 2 794 969 A1 describes an adhesive bandage comprising a support with an adhesive surface, a sterile pad arranged on said adhesive surface and a protective film covering the adhesive surface and the sterile pad, which is formed by a first and a second tearaway tab. The first tab is folded back on itself along a folding line, outside the sterile pad zone, to define a first flap applied on the adhesive surface from a first end of the support up to the folding line and covering entirely the sterile pad, and a second flap forming a tearing lead for the first flap and extending over said first flap beyond the support first end. The second tab comprises a third flap applied on the adhesive surface from the second end of the support up to the folding line, and a fourth flap forming a tearing lead for the third flap which extends over the first tab from the folding line beyond the support first end.

### DISCLOSURE OF THE INVENTION

### PROBLEMS TO BE SOLVED BY THE INVENTION

The present invention addresses the above-described problems of the conventional adhesive patches. To that end, it is an object of the present invention to provide a production method of an adhesive patch such as poultice-type patch that can be easily applied not only by non-disabled people, but also by aged people and patients with decreased grip strength, by facilitating the sequence of actions required to apply the patch, from the removal of the liner to the application of the patch. The adhesive patch produced by the method of the present invention can be applied in a safe and hygienic manner since the drug-containing matrix does not come into contact with hands during the application. It is another object of the present invention to provide a production method of an adhesive patch such as poultice-type patch that is suitable for mass production.

### MEANS FOR SOLVING THE PROBLEMS

One essential aspect of the present invention to solve the aforementioned problems is provided by the following invention according to claim 1:
A method for producing a adhesive patch having a multilayer structure comprising a backing, an adhesive drug-containing matrix that is spread substantially entirely over one surface of the backing, and a liner that adheres to the drug-containing matrix surface, wherein
(a) the liner adhering to the drug-containing matrix surface comprises first and second liners;
(b) the first liner is folded at the middle thereof so that it is divided by the fold into first and second sections that together form a V-shaped liner, the first section adhering to the drug-containing matrix surface with the fold arranged closer to the middle of the drug-containing matrix; and
(c) the second liner adheres to the remaining part of the drug-containing matrix surface with one end of the second liner covering the fold of the V-shaped first liner,
the method comprising the steps of: allowing a tubular liner to adhere to at least half of the surface of the drug-containing matrix spread over the backing; and cutting the tubular liner in half to form the V-shaped first liner folded at the middle thereof.

More specifically, the invention according to claim 2 is the method according to claim 1, wherein the second liner that adheres to the remaining part of the drug-containing matrix surface is a sheet-like liner and the end of the second liner covering the fold of the first liner is shaped as a straight line, a curved line, such as a wave shape or a mountain shape, or a combination thereof.

Another specific invention according to claim 3 is the method according to claim 1, wherein the second liner that adheres to the remaining part of the drug-containing matrix surface is a tubular liner, the tubular liner being cut in half to form a folded V-shaped second liner having a fold covering the fold of the first liner.

A more specific invention according to claim 4 is the above-described method, wherein the first liner and/or the second liner comprises a plastic film formed of cast polypropylene, oriented polypropylene, polyethylene terephthalate, polybutylene terephthalate, polyethylene, polyester, polyurethane, polyvinyl chloride or polystyrene; paper, synthetic paper, synthetic resin or a composite film formed of a laminate thereof; or a composite film formed of a laminate of aluminum foil or aluminum-deposited film.

Inventions according to claims 5 and 6 are the above-described method, wherein the first liner and/or the second liner is treated with silicone or embossed.

As described above, the adhesive patch produced by the method of the present invention has the following features: Two films, the first liner and the second liner, adhere to the, with the film being a tubular liner. At least first liner that adheres to the drug-containing matrix surface is formed into V-shape by cutting the tubular liner in half. The other surface serves as a tab. The second or upper liner also includes a tab.

### EFFECT OF THE INVENTION

As described above, the adhesive patch produced by the method of the present invention includes the tab on the second or upper liner. The user can easily pinch the tab with his fingers and peel the upper second liner. This can be done in a safe and simple manner and requires only a weak force.
The user then pinches with his fingers the tab of the V-shaped lower first liner laminated to the backing and pulls the tab so that it slides outward. This causes the exposed drug-containing matrix to spontaneously attach to the application site.

Thus, by using the adhesive patch produced by the method of the present invention, the user does not need to exert a substantial force to form a part from which to start peeling the liner upon removal of the film. The adhesive patch produced by the method of the present invention therefore offers an advantage in that it enables people who cannot exert much physical power, such as aged people, to peel the liner. The adhesive patch thus facilitates the sequence of actions required to apply the patch, from the removal of the liner to the application of the patch with one hand.

Still another advantage of the adhesive patch is that the stickiness and the skin followability of the drug-containing matrix are not affected since the drug-containing matrix does not come into contact with the hands or fingers during the sequence of actions from the removal of the liner to the application of the patch to the application site. Since the drug-containing matrix is free of contamination and maintains good hygiene, the adhesive patch is safe for application to the damaged skin.
Yet another advantage of the adhesive patch is that the patch does not cause sticky fingers or hands, so that good hygiene can be maintained during the application of the patch and the user does not need to wash hands after application of the patch.

Another advantage is that the use of the tubular liner ensures stable product quality and eliminates the need to pay special attention to the production process upon occurrences of meandering film or upon stopping and restarting of the machine operation. Thus, the method of the present invention is suitable for mass production and enables significant cost reduction.

### BEST MODE FOR CARRYING OUT THE INVENTION

The liner for use in the method of the present invention may be a resin film formed of such as cast polypropylene (CPP), oriented polypropylene (OPP), polyethylene terephthalate (PET), polybutylene terephthalate (PBT), polyethylene, polyester, polyurethane, polyvinyl chloride and polystyrene, paper, synthetic paper, synthetic resin or a composite film formed of a laminate thereof, an aluminum foil or aluminum-deposited film laminated with any of the above-described materials, or each or a composite of the above-described materials that is treated with silicone, is embossed, has a printed pattern or is colored.

The tubular liner material for use in the present invention can be produced by any suitable film-forming method including, but not limited to, inflation method, T-die method, calendering and solution casting. However, these methods, other than the inflation method, require a tubing process to form the tubular film. Thus, the material must be laminated with a seal layer for heat-sealing if it is not sealable to itself.

The liner has a thickness in the range of 10 to 200 µm, and preferably in the range of 25 to 75 µm. The liner with a thickness of less than 10 µm is too thin to be properly held by the fingers and tends to wrinkle during the production of the adhesive patches. The liner with a thickness of more than 200 µm is difficult to cut during the production of the adhesive patches, which adds to the cost of the adhesive patches. Such liner is also expensive and is therefore unfavorable in terms of cost.

According to the present invention, the liner is preferably embossed to prevent slipping of the fingers upon removal of the film. The liner may be embossed either entirely or partially (for example, in the tab).

The liner may be embossed with any pattern that provides a good grip for the fingers, such as diamond pattern, lattice pattern, hexagonal pattern, wave pattern and various other patterns.

In the inflation method, the liner is embossed in the form of tubular original film. In other film-forming methods, the liner may be embossed either before or after the tubing process. However, when the liner is to be embossed with a pattern having a relatively large profile, it must be embossed after the tubing process since sealing will otherwise be difficult.

To clearly indicate to the user the method of peeling the liner, characters, arrows, symbols, illustrations and other indicative marks may be provided on either or both of the first and second liners. Either or both of the first and second liners may also be colored.

The part that serves as the tab of the second liner, that is, the overlap (laminated part) of the upper liner and the lower V-shaped first liner, is preferably from about 10 to 30mm wide, and more preferably from 15 to 25mm wide. The tab that is less than 10mm wide is not desirable since it can hardly be held by the fingers and cause meandering during production, thus leading to a decreased workability. It also leads to a decreased yield of the adhesive patches. The tab that is more than 30mm wide is not desirable, either, since the laminated part not only adds to the cost, but also tends to roll up upon packaging, though the wider tab is easier to hold with the fingers.

Preferably, the laminated part of the upper second liner and the lower V-shaped first liner may contain characters, arrows, symbols, illustrations and other indicative marks that indicate the end to the user. The laminated part may also be colored for the same purpose. To further distinguish the tab, the tab may be embossed differently from the remaining part of the film.

The upper second liner and the lower V-shaped first liner may adhere to the drug-containing matrix at any area ratio. Preferably, the ratio of the area of the drug-containing matrix to which the upper second liner adheres, to the area to which the lower V-shaped first liner adheres is in the range of 3:1 to 1:3, and more preferably in the range of 1:1 to 2:1.

If the ratio of the area to which the upper second liner adheres, to the area to which the lower V-shaped first liner adheres is smaller than 3:1, then the user needs to take special care to prevent the adhesive patch from falling during the application. Specifically, in applying the adhesive patch to the application site, the user first peels the upper second liner and attaches the drug-containing matrix to the application site. The user then peels the upper V-shaped first liner by slightly sliding the first liner with his fingers. During this, the user needs to keep the adhesive patch from falling by pressing the adhesive patch against the application site with the other hand. Furthermore, the lower V-shaped liner that adheres to a larger area leads to an increased cost.

Likewise, if the ratio of the area to which the upper second liner adheres, to the area to which the lower V-shaped first liner adheres is larger than 1:3, then the user must peel the liners slowly and carefully to keep the drug-containing matrix from sticking to itself during the removal of the upper second liner. In addition, the distance between the tab of the upper second liner and the tab of the lower V-shaped first liner becomes so small that the user, in an attempt to peel the upper second liner, may pinch both of the tabs of the upper and the lower liners at once.

The cut edge of the tab of the upper second liner may be shaped into any shape including, but not limited to, straight line, wave shape and mountain shape. While the end of the tab of the upper second liner may be shaped into any shape ranging from a straight line to a gently curved line, pointy shapes are not preferred since the user may cut his finger with the edge of the liner.

Likewise, the cut edge of the tab of the lower V-shaped first liner may also be shaped into any shape including, but not limited to, straight line, wave shape and mountain shape. While the end of the tab of the lower V-shaped first liner may be shaped into any shape ranging from a straight line to a gently curved line, pointy shapes are not preferred since the user may cut his finger with the edge of the liner. Pointy shapes are not preferred also because the pointy edges can interfere with the adhering of the lower V-shaped liner to the drug-containing matrix during the production of the adhesive patches, resulting in a decreased yield of the adhesive patches.

The backing for use in the present invention may be woven fabric, non-woven fabric or a laminate thereof and may or may not be stretchable.

Specific examples of the material for the backing include natural fibers, including bast fibers, such as paper, cotton, hemp and jute, cellulose fibers, such as leaf fibers (such as manila hemp), animal fibers, such as wool, and protein fibers, such as silk fibers and feather fibers; regenerated fibers, including regenerated cellulose fibers, such as rayon and cuprammonium rayon, and regenerated protein fibers; semi-synthetic fibers, including cellulose acetate fibers and promix; nylon alamide fibers; polyethylene terephthalate fibers; polyester fibers; acrylic fibers; polyolefin fibers, including polyethylene fibers and polypropylene fibers; polyvinyl alcohol fibers; polyvinyl chloride fibers; polyvinylidene chloride fibers; polyvinyl chloride-based fibers; polyurethane fibers; polyoxymethylene fibers; polytetrafluoroethylene fibers; poly(p-phenylene benz-bisthiazole) fibers; and polyimide fibers. These fibers may be used either individually or as a composite fiber to make a woven or non-woven fabric for use in the present invention.

The backing is properly selected from the above-described materials based on the tensile strength, thickness and stretchability required for a particular application site, as well as drug transfer to the backing.

The drug-containing matrix for use in the adhesive patch contains a base and a drug and is suitable for adhesive patches used, for example, as external poultice-type patches. The drug-containing matrix also contains water to enhance the effect of the drug on the skin. The drug-containing matrix has stickiness and does not soften at room temperature or a higher temperature so that it retains moderate cohesiveness that prevents the drug-containing matrix from remaining on the skin.

A thickener may be used in the drug-containing matrix. The thickener serves to stably maintain the water content of the drug-containing matrix at 30% to 80% and preferably shows water retention. Specific examples of the thickener include water-soluble polymers, including natural polymers, such as plant-based polymers (such as guar gum, locust bean gum, carrageenan, alginic acid, sodium alginate, agar, gum acacia, tragacanth gum, karaya gum, pectin and starch), microorganism-based polymers (such as xanthan gum and acacia gum), and animal-based polymers (such as gelatin and collagen); semi-synthetic polymers, such as cellulose-based polymers (such as methyl cellulose, ethyl cellulose, hydroxyethyl cellulose and carboxymethylcellulose sodium), and starch-based polymers (such as soluble starch, carboxymethyl starch and dialdehyde starch); and synthetic polymers, such as vinyl-based polymers (such as polyvinyl alcohol, polyvinylpyrrolidone and polyvinylmethacrylate), acryl-based polymers (such as polyacrylic acid and sodium polyacrylate), polyoxyethylene oxides, and methylvinylether/maleic anhydride copolymers.

Of these, sodium polyacrylate is particularly preferred because of its high gel strength and good water retention. Sodium polyacrylate preferably has an average degree of polymerization of 20,000 to 70,000. Sodium polyacrylate having an average degree of polymerization of less than 20,000 exhibits less thickening effect, failing to provide sufficient gel strength. Conversely, sodium polyacrylate exhibits too high a thickening effect and leads to decreased workability when it has an average degree of polymerization of higher than 70,000. An elastic gel having even higher gel strength can be obtained by using sodium polyacrylate in combination with two or more of the above-described water-soluble polymers since sodium polyacrylate, a highly ionic polymer, forms a polymer complex with the water-soluble polymers.

A humectant may be used in the drug-containing matrix. Examples include polyols, such as glycerin, propylene glycol and sorbitol. In addition, a filler, such as kaolin, zinc oxide, talc, titanium, bentonite, aluminum silicate, titanium oxide, zinc oxide, aluminum metasilicate, calcium sulfate and calcium phosphate, may be added. A solubilizer or an absorption enhancer, such as propylene carbonate, crotamiton, 1-menthol, mint oil, limonene and diisopropyl adipate, may also be added. A drug-activity enhancer, such as methyl salicylate, glycol salicylate, 1-menthol, thymol, mint oil, nonanoic acid vanillylamide and capsicum extract, may also be added. When necessary, a stabilizer, an antioxidant or an emulsifier may also be added.

When necessary, the drug-containing matrix may also contain a crosslinking agent or a polymerization agent to strengthen the drug-containing matrix and to impart the water retention property to the drug-containing matrix. The crosslinking agent or the polymerization agent may be properly selected depending on the type of the thickener used. For example, when the thickener is polyacrylic acid or a polyacrylate, the crosslinking agent is preferably a polyvalent metal compound, including compounds having at least two epoxy groups in their molecules; inorganic salts, such as hydrochlorides, sulfates, phosphates and carbonates of Ca, Mg and Al; organic salts, such as citrates, tartrates, gluconates and stearates; oxides, such as zinc oxide and silicic anhydride; and hydroxides, such as aluminum hydroxide and magnesium hydroxide.

When the thickener is polyvinyl alcohol, the crosslinking agent or the polymerization agent is preferably adipic acid, thioglycol acid, epoxy compounds (epichlorohydrin), aldehydes, N-methylol compounds or a complex of compounds, such as Al, Ti, Zr, Sn, V, Cu, B and Cr.

When the thickener is polyvinylpyrrolidone, the crosslinking agent or the polymerization agent is preferably a methylvinylether/maleic anhydride copolymer, or a polyacid compound or an alkali metal salt thereof (such as polyacrylic acid, tannic acid and derivatives thereof).

When the thickener is polyethylene oxide, the crosslinking agent or the polymerization agent is preferably a peroxide or polysulfone azide. When the thickener is methylvinylether/maleic anhydride copolymer, the crosslinking agent or the polymerization agent is preferably a polyfunctional hydroxyl compound, polyamine, iodine, gelatin, polyvinylpyrrolidone, iron, mercury or lead salt.

When the thickener is gelatin, the crosslinking agent or the polymerization agent is preferably an aldehyde, such as formaldehyde, glutaraldehyde and dialdehyde starch, a diepoxide, such as gluoxal and butadieneoxide, a diketone, such as divinyl ketone, or a diisocyanate. When the thickener is sodium polyacrylate, the crosslinking agent added is preferably a polyvalent metal salt, such as lithium hydroxide, zinc hydroxide, aluminum hydroxide and sodium borate. Zinc salts and aluminum salts promote crosslinking reactions and are thus particularly preferred.

The polyvalent metal salt used as the crosslinking agent is preferably used at a concentration of 0.5 to 1.5 equivalents relative to 1 equivalent of the thickener (or water-soluble polymer). The polyvalent metal salt used at a concentration of less than 0.5 equivalents does not sufficiently promote the crosslinking reaction, resulting in a low gel strength, whereas the polyvalent metal salt used at a concentration of higher than 1.5 equivalents excessively accelerates the crosslinking reaction, resulting in non-uniform gelation and, thus, decreased workability.

A poultice-type patch is required to stick firmly to the skin, enhance absorption of the active ingredient by the skin, and contain as much water as possible. The water present in the drug-containing matrix removes heat from the skin and causes coolness as it evaporates. The water molecules evaporating from inside the matrix hydrate the stratum corneum and thereby promote the drug absorption. Requirements for the drug-containing matrix include the following: it should not soften near room temperature; it should not cause pain or remain on the skin when the adhesive patch is peeled; and it should not cause stickiness.

To meet the above-described requirements, the drug-containing matrix contains 5 to 20 wt%, preferably 10 to 15 wt% of the thickener, 5 to 40 wt% of the humectant, 20 wt% or less of the filler, 10 to 80 wt% of water, 0 to 8 wt% of the solubilizer, and 5 wt% or less, preferably 0.5 to 5 wt% of the drug.

The drug used as an active ingredient in the adhesive patch produced by the method of the present invention can be selected from a variety of drugs to suit the intended application. Analgesic and anti-inflammatory agents that can be used in the adhesive patch include indomethacin, ketoprofen, flurbiprofen, ibuprofen, felbinac, glycol salicylate, methyl salicylate, glycyrrhizinic acid, dipotassium glycyrrhizinate and β-glycyrrhizinic acid.

Blood circulation-promoting agents that can be used in the adhesive patch include tocopherol acetate, capsicum extract, capsaicin, nonanoic acid vanillylamide, benzyl nicotinate and benzyl alcohol. Antiallergic agents that can be used in the adhesive patch include diphenhydramine hydrochloride and chlorpheniramine maleate. Locally stimulating agents that can be used in the adhesive patch include 1-menthol, camphor, mint oil and eucalyptus oil. Local anesthetic agents that can be used in the adhesive patch include lidocaine, benzocaine, dibucaine and tetracaine. The drugs for use in the adhesive patch are not limited to those described above and may be used in combination of two or more as desired.

The amount of the drug used in the drug-containing matrix is properly determined depending on the type and the intended application of the adhesive patch such as poultice-type patch so that the drug is delivered to the application site at a predetermined effective dose when the adhesive patch is applied to the patients.

### EXAMPLES

The present invention will now be described by way of several embodiments serving as concrete examples with reference to the accompanying drawings.

### [Embodiment 1]

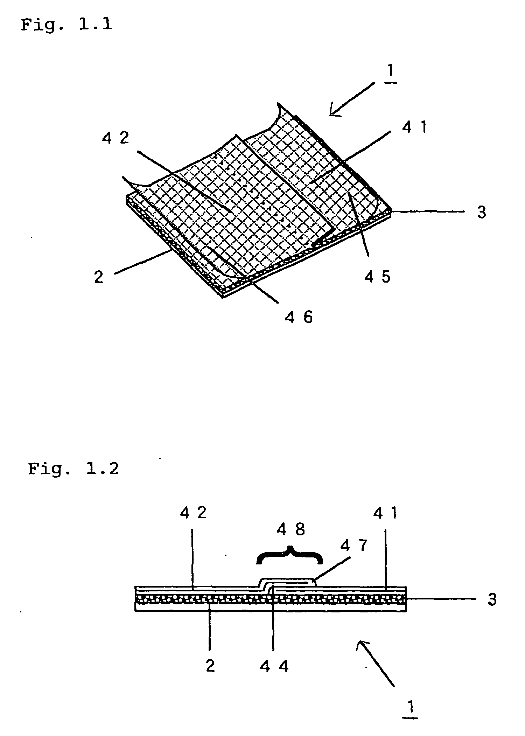

Shown in Fig. 1 is an adhesive patch, such as a poultice-type patch, of Embodiment 1 produced in accordance with one example of the present invention. Fig. 1.1 is a perspective view of the adhesive patch and Fig. 1.2 is a side view thereof.

The poultice-type patch 1 or the adhesive patch of example shown in Fig. 1 is essentially constructed as a laminate comprising a backing 2 formed of a stretchable non-woven fabric, a drug-containing matrix 3 spread over the substantially entire surface of the backing 2, and a pair of liners 41 and 42 that adheres to the surface of the drug-containing matrix 3.

Of the two liners that adhere to the surface of the drug-containing matrix 3 of the poultice-type patch 1, the lower liner or first liner 41 is folded at the middle thereof so that it is divided by the fold into two sections that together form a V-shape. One of the two sections of the V-shaped first liner 41 adheres to the drug-containing matrix surface from one end of the matrix so that the fold 44 is arranged closer to the middle of the drug-containing matrix. The other of the two sections of the V-shaped liner serves as a tab 45.

Of the two liners that adhere to the surface of the drug-containing matrix, the upper second liner 42 is folded at the middle thereof so that it is divided by the fold into two sections that together form a V-shaped liner. One of the two sections of the V-shaped second liner 42 adheres to the remaining part of the drug-containing matrix surface with the fold 47 of the second liner overlaying and covering the fold 44 of the lower V-shaped first liner 41. The other of the two sections of the V-shaped second liner 42 serves as a tab 46 to be held by the user's fingers.

In this example, the drug-containing matrix 3 spread over the substantially entire surface of the backing 2 is formed of a material such as sodium polyacrylate and contains, along with water, a drug such as felbinac, an anti-inflammatory/analgesic agent that serves as an active ingredient.

In the present example, the following films are used as the liners 41 and 42 that adhere to the surface of the drug-containing matrix: The upper second liner 42 that comprises a 30 to 50µm-thick tubular liner formed of cast polypropylene and embossed with a diamond pattern and adheres to substantially half of the matrix surface to form an overlap 48, and the lower first liner 41 that adheres to the remaining half of the matrix surface and comprises a 30 to 50µm-thick tubular liner formed of cast polypropylene and embossed with a diamond pattern. The first liner 41 and the second liner 42 are each formed as a V-shaped liner comprising a tubular liner cut in half.

In the present example, the ratio of the area of the drug-containing matrix 3 to which the upper second liner 42 adheres, to the area to which the lower V-shaped first liner 41 adheres is 1:1. The overlap 48 in which the upper second liner 42 is laminated to the first liner is preferably 15 to 25 mm in width.

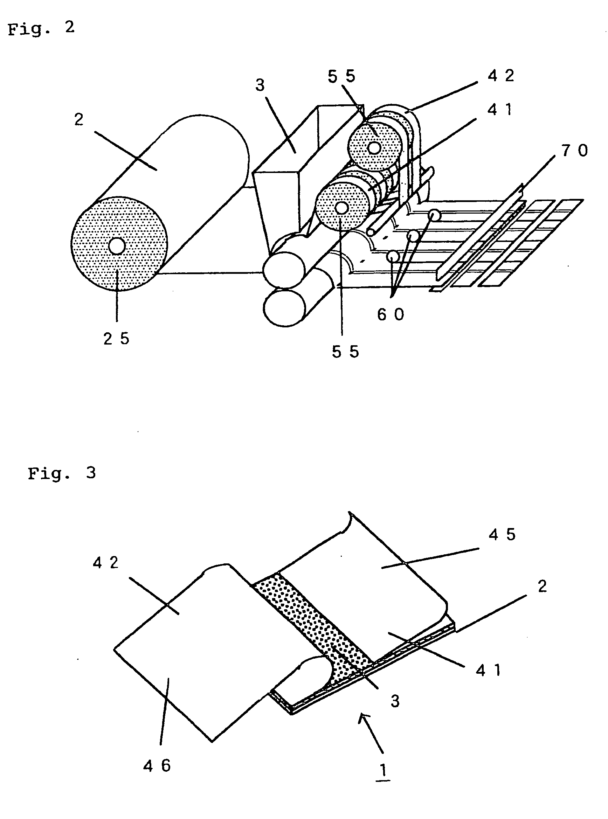

The steps of the production method of the poultice-type patch 1 or the adhesive patch based on this example are shown in Fig. 2 in a schematic diagram.
Specifically, the adhesive drug-containing matrix 3 is spread onto the backing 2 as the backing is drawn from a backing roll 25. The surface of the drug-containing matrix is then laminated with a tubular liner to make the upper second liner 42 and a tubular liner to make the lower first liner 41 as the tubular liners are drawn from respective liner rolls 55. The resulting laminate is cut into strips 60 so that each tubular liner is cut in half. The strips are then cut into pieces with a predetermined size 70.

How the poultice-type patch 1 that is constructed based on the above-described example is applied to an application site will now be described.

Fig. 3 shows in a schematic perspective view the manner in which the upper second liner 42 of the poultice-type patch 1 of the above-described example is peeled.
Fig. 4 shows in a schematic side view the manner in which the drug-containing matrix 3 having substantially half of its surface exposed by peeling the upper second liner 42 of the poultice-type patch 1 of the above-described example is attached to the application site.
Fig. 5 shows in a schematic side view the manner in which the drug-containing matrix 3, following the procedure of Fig. 4, is continuously attached to the application site as the remaining lower V-shaped first liner 41 is peeled off.

Specifically, the user first pinches with his fingers (not shown) the tab 46 of the upper second liner 42 of the poultice-type patch 1 and peels the upper second liner 42 as shown in Fig. 3. Once the second liner 42 is completely peeled, (half of) the surface of the drug-containing matrix 3 to which the second liner 42 has adhered.is exposed.

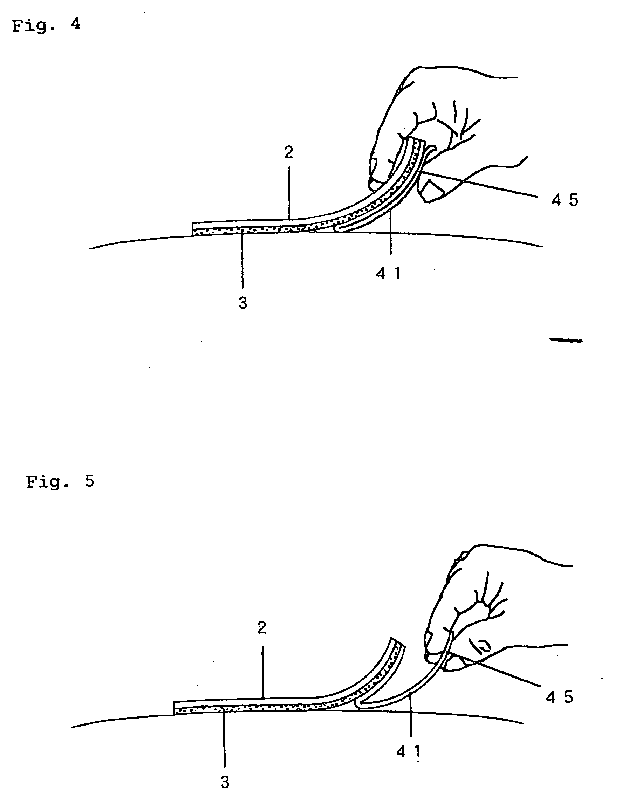

Subsequently, the user attaches the exposed surface of the drug-containing matrix to the application site and then holds the backing 2 together with the tab 45 of the lower V-shaped first liner 41, as shown in Fig. 4.

As shown in Fig. 5, the user then slides outward one of the fingers held against the tab 45 of the lower V-shaped first liner 41. The peeling of the first liner 41 causes the exposed drug-containing matrix 3 to spontaneously attach to the application site.

As a result, the series of actions required to apply the patch, starting from the removal of the liner and ending in the application of the poultice-type patch, can be readily carried out in a single sequence. In addition, the user can apply the poultice-type patch in a safe and hygienic manner without his hands coming into contact with the drug-containing matrix during the application.

### [Embodiment 2]

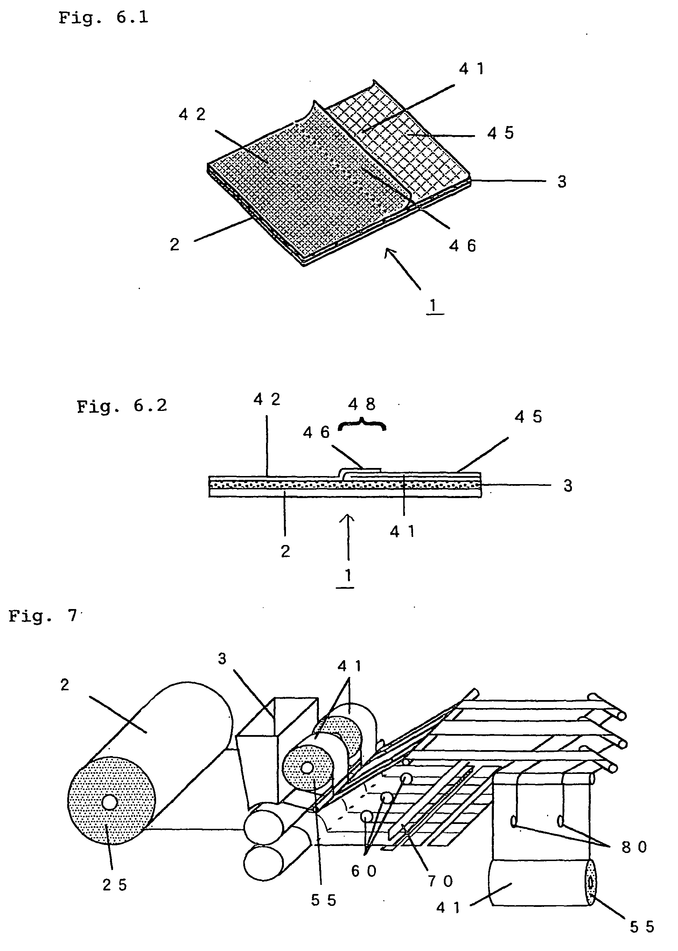

Shown in Fig. 6 is an adhesive patch, such as a poultice-type patch, of Embodiment 2 produced in accordance with another example of the present invention. Fig. 6.1 is a perspective view of the adhesive patch and Fig. 6.2 is a side view thereof.
The numerals in Fig. 6 denote the same elements as in Fig. 1.

In the present example, the poultice-type patch 1 is similar to the poultice-type patch of the above-described example in that it is constructed as a laminate comprising a backing 2, a drug-containing matrix 3 spread over the entire surface of the backing 2, and a pair of liners 41 and 42 that adheres to the surface of the drug-containing matrix 3. In the present example, however, the drug-containing matrix 3 spread over the substantially entire surface of the backing 2 is formed of sodium polyacrylate. The drug-containing matrix 3 also contains, along with water, a drug such as indomethacin, an anti-inflammatory/analgesic agent that serves as an active ingredient.

In this example, the two liners 41, 42 are as follows: The upper second liner 42 is a 30 to 50µm-thick cast polypropylene film embossed with a hexagonal pattern.

The lower first liner 41 is a tubular liner cut in half. The film is formed of cast polypropylene and embossed with a diamond pattern.

In the present example, the ratio of the area of the drug-containing matrix 3 to which the upper second liner 42 adheres, to the area to which the lower V-shaped first liner 41 adheres is also 1:1. The overlap 48 in which the upper second liner 42 is laminated to the first liner is preferably 15 to 25 mm in width.

The steps of the production method of the poultice-type patch 1 or the adhesive patch provided in this example are shown in Fig. 7 in a schematic diagram.
Specifically, the adhesive drug-containing matrix 3 is spread onto the backing 2 as the backing is drawn from a backing roll 25. The surface of the drug-containing matrix is then laminated with a tubular liner to make the upper second liner 42 and a tubular liner to make the lower first liner 41 that are drawn from a second liner roll 55 and a first liner roll 55, respectively. The resulting laminate is cut into strips 60 so that each tubular liner is cut in half. The strips are then cut into pieces with a predetermined size 70.

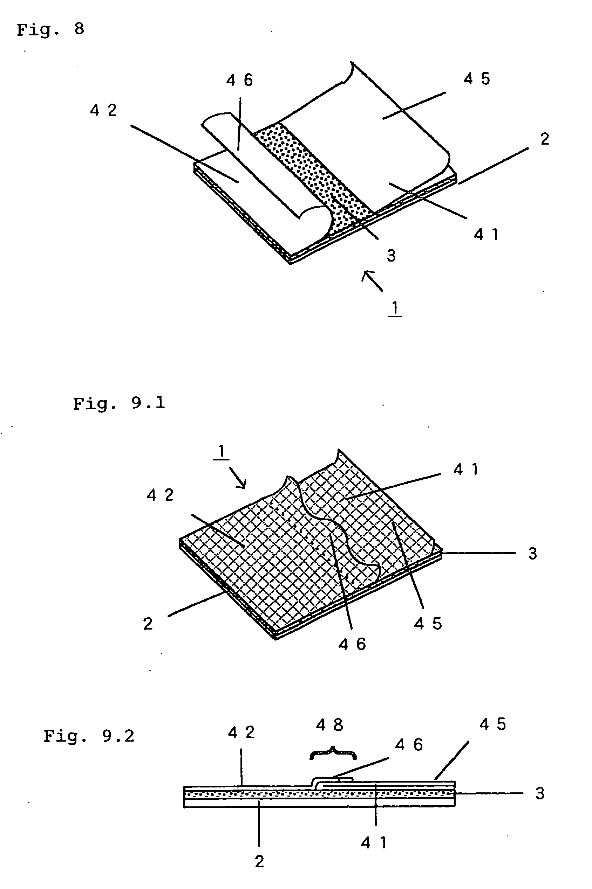

In the present example, the poultice-type patch 1 is applied to the application site in the same manner as in the above-described example. The manner in which the upper second liner 42 of the poultice-type patch 1 based on the above-described example of the present invention is peeled is shown in a schematic perspective view in Fig. 8.

### [Embodiment 3]

Shown in Fig. 9 is an adhesive patch, such as a poultice-type patch, of Embodiment 3 produced in accordance with still another example of the present invention. Fig. 9.1 is a perspective view of the adhesive patch and Fig. 9.2 is a side view thereof.
The numerals in Fig. 9 denote the same elements as in Fig. 1.

In the present example, the poultice-type patch 1 is similar to the poultice-type patch of the above-described example in that it is constructed as a laminate comprising a backing 2, a drug-containing matrix 3 spread over the entire surface of the backing 2, and a pair of liners 41, 42 that adheres to the surface of the drug-containing matrix 3. The backing, the drug-containing matrix, the ratio of the area of the drug-containing matrix to which the upper second liner 42 adheres, to the area to which the lower first liner 42 adheres and the size of each tab are the same as those described in Example 2 and will not be described again.

The poultice-type patch 1 of Embodiment 3 differs from the poultice-type patch 1 of Embodiment 2 in that the upper second liner 42 and the lower first liner 41 are each a liner obtained by laminating a 25 to 38µm-thick polyethylene terephthalate film with a 10 to 30µm-thick low-density polyethylene film, treating the laminate with silicone and embossing the laminate with a diamond pattern. Another difference is that the cut edge of the tab 46 of the upper second liner 41 has a wave shape.

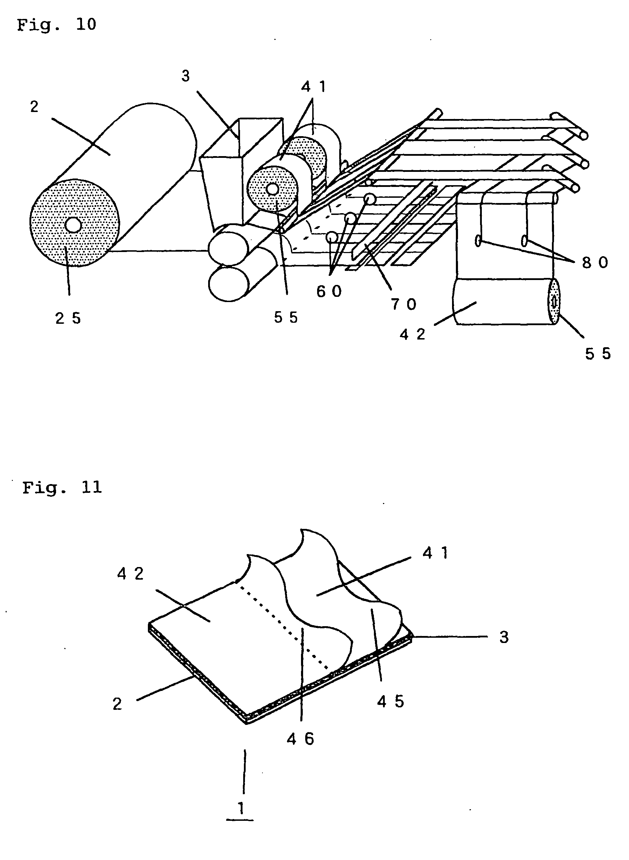

The steps of the production method of the poultice-type patch 1 or the adhesive patch provided in this example are shown in Fig. 10 in a schematic diagram.
Specifically, the adhesive drug-containing matrix 3 is spread onto the backing 2 as the backing is drawn from a backing roll 25. The surface of the drug-containing matrix is then laminated with a tubular liner to make the upper second liner 42 and a tubular liner to make the lower first liner 41 that are drawn from a second liner roll 55 and a first liner roll 55, respectively. The resulting laminate is cut into strips 60 so that each tubular liner is cut in half. The strips are then cut into pieces with a predetermined size 70.
One end of the upper second liner 42 is cut into a wave shape 80.

Since the adhesive patch of Embodiment 3 having the above-described construction is used in the same manner as the adhesive patch of Embodiment 2, how the adhesive patch is used will not be described again.
However, when the upper second liner 42 and the lower first liner 41 are each formed of the 25 to 38µm-thick polyethylene terephthalate film that is harder to bend than the cast polypropylene film, the user can inadvertently injure his fingers especially with the upper second liner 42 while trying to hold the tabs of the upper second liner and the lower first liner.
To prevent this, the cut edges of the tabs are shaped into a wave shape to significantly reduce the risk of cutting fingers.

It should be understood that the edge of the tabs may be shaped not only as the wave shape employed in this example, but also as a mountain shaped curved line or a combination thereof.
Such variations are shown in Figs. 11 and 12.

### INDUSTRIAL APPLICABILITY

As set forth, the adhesive patch produced by the method of the present invention uses two liners, first and second liners, that adhere to the surface of the drug-containing matrix and each include a tab that the user can easily pinch with his fingers to peel the films. This construction not only allows the user to remove the liners in a safe and simple manner by exerting only a weak force, but also enables the user to easily perform, with a single hand, the sequence of actions required to apply the patch, from the removal of the liners to the application of the patch.

According to the adhesive patch produced by the method of the present invention, the stickiness and the skin followability of the drug-containing matrix are not affected since the drug-containing matrix does not come into contact with the hands or fingers during the sequence of actions from the removal of the liner to the application of the patch to the application site. Since the drug-containing matrix is free of contamination, the adhesive patch can be applied in a highly hygienic manner and is therefore of significant industrial importance.

### BRIEF DESCRIPTION OF THE DRAWINGS

Fig. 1.1 is a perspective view showing a poultice-type patch 1 of Embodiment 1 produced in accordance with one example of the present invention.
Fig. 1.2 is a side view of the poultice-type patch 1 of Embodiment 1.
Fig. 2 is a schematic diagram showing the steps of the production method of the poultice-type patch or the adhesive patch based on the example above.
Fig. 3 is a schematic perspective view showing the manner in which the upper second liner of the poultice-type patch produced by the method of the above example of the present invention is peeled.
Fig. 4 is a schematic side view showing the manner in which the upper second liner of the poultice-type patch produced by the method of the above example of the present invention is peeled to expose substantially half of the surface of the drug-containing matrix to apply the patch to the application site.
Fig. 5 is a schematic side view showing the manner in which the drug-containing matrix, following the procedure of Fig. 3, is continuously attached to the application site as the remaining lower V-shaped first liner is peeled off.
Fig. 6.1 is a perspective view showing a poultice-type patch of Embodiment 2 produced in accordance with another example of the present invention.
Fig. 6.2 is a side view of the poultice-type patch 1 of Embodiment 2.

Fig. 7 is a schematic diagram showing the steps of the production method of the poultice-type patch or the adhesive patch based on another example of the present invention.
Fig. 8 is a schematic perspective view showing the manner in which the upper second liner of the poultice-type patch produced by the method of another example of the present invention is peeled.
Fig. 9.1 is a perspective view showing a poultice-type patch of Embodiment 3 produced in accordance with still another example of the present invention.
Fig. 9.2 is a side view of the poultice-type patch of Embodiment 3.
Fig. 10 is a schematic diagram showing the steps of the production method of the poultice-type patch or the adhesive patch based on still another example of the present invention.
Fig. 11 is a diagram showing one variation of the edge of the tabs.
Fig. 12 is a diagram showing another variation of the edge of the tabs.
Fig. 13.1 is a perspective view showing a conventional poultice-type patch.
Fig. 13.2 is a side view of the conventional poultice-type patch.
Fig. 14 is a schematic diagram showing the steps of the production method of the conventional poultice-type patch.
Fig. 15.1 is a perspective view showing another conventional poultice-type patch.
Fig. 15.2 is a perspective view showing the conventional poultice-type patch that has its peelable paper sheet removed.
Fig. 15.3 is a side view of the conventional poultice-type patch.
Fig. 16 is a schematic diagram showing the steps of the production method of another conventional poultice-type patch.

### DESCRIPTION OF REFERENCE NUMERAL

- 1: poultice-type patch (adhesive patch)
- 2: backing
- 3: drug-containing matrix
- 4: liner
- 41: first liner
- 42: second liner
- 44: fold
- 45: tab
- 46: tab
- 48: overlap

## Claims

1. A method for producing an adhesive patch (1) having a multilayer structure comprising a backing (2), an adhesive drug-containing matrix (3) that is spread substantially entirely over one surface of the backing (2), and a liner that adheres to the drug-containing matrix surface, wherein
(a) the liner adhering to the drug-containing matrix (3) surface comprises first and second liners (41, 42);
(b) the first liner (41) is folded at a middle thereof so that it is divided by the fold (44) into first and second sections that together form a V-shaped liner, the first section adhering to the drug-containing matrix (3) surface with the fold (44) arranged closer to the middle of the drug-containing matrix (3); and
(c) the second liner (42) adheres to the remaining part of the drug-containing matrix (3) surface with one end of the second liner (42) covering the fold (44) of the V-shaped first liner (41),
**characterized in that** the method comprises the steps of: allowing a tubular liner to adhere to at least half of the surface of the drug-containing matrix (3) spread over the backing (2); and cutting the tubular liner in half to form the V-shaped first liner (41) folded at the middle thereof.

2. The method for producing an adhesive patch (1) according to claim 1, wherein the second liner (42) that adheres to the remaining part of the drug-containing matrix (3) surface is a sheet-like liner and the end of the second liner (42) covering the fold (44) of the first liner (41) is shaped as a straight line, a curved line, such as a wave shape or a mountain shape, or a combination thereof.

3. The method for producing an adhesive patch (1) according to claim 1, wherein the second liner (42) that adheres to the remaining part of the drug-containing matrix (3) surface is a tubular liner, the tubular liner being cut in half to form a folded V-shaped second liner (42) having a fold covering the fold (44) of the first liner.

4. The method for producing an adhesive patch (1) according to any of claims 1 to 3, wherein the first liner (41) and/or the second liner (42) comprises a plastic film formed of cast polypropylene, oriented polypropylene, polyethylene terephthalate, polybutylene terephthalate, polyethylene, polyester, polyurethane, polyvinyl chloride or polystyrene; paper, synthetic paper, synthetic resin or a composite film formed of a laminate thereof; or a composite film formed of a laminate of aluminum foil or aluminum-deposited film.

5. The method for producing an adhesive patch (1) according to any of claims 1 to 4, wherein the first liner (41) and/or the second liner (42) is treated with silicone.

6. The method for producing an adhesive patch (1) according to any of claims 1 to 5, wherein the first liner (41) and/or the second liner (42) is embossed.

## Patentansprüche

1. Verfahren zur Herstellung eines Heftpflasters (1) mit einer Multischichtstruktur, umfassend einen Träger (2), eine wirkstoffhaltige Klebematrix (3), die im wesentlichen über die ganze Oberfläche des Trägers (2) verteilt ist, und ein Trennpapier, das an der wirkstoffhaltigen Matrixoberfläche haftet, wobei
(a) das an der Oberfläche der wirkstoffhaltigen Matrix (3) haftende Trennpapier ein erstes und zweites Trennpapier (41, 42) umfasst;
(b) das erste Trennpapier (41) in einer Mitte davon gefaltet ist, sodass es durch die Faltung (44) in erste und zweite Bereiche geteilt wird, die gemeinsam ein V-förmiges Trennpapier bilden, wobei bei dem ersten Bereich, der an der Oberfläche der wirkstoffhaltigen Matrix (3) haftet, die Faltung (44) näher an der Mitte der wirkstoffhaltigen Matrix (3) angeordnet ist; und
(c) das zweite Trennpapier (42) an dem verbleibenden Teil der Oberfläche der wirkstoffhaltigen Matrix (3) haftet, wobei ein Ende des zweiten Trennpapiers (42) die Faltung (44) des V-förmigen ersten Trennpapiers (41) bedeckt,
**dadurch** charakterisiert, dass das Verfahren die folgenden Schritte umfasst: Haftenlassen eines schlauchförmigen Trennpapiers an mindestens der Hälfte der Oberfläche der wirkstoffhaltigen Matrix (3), die über den Träger (2) verteilt ist; und Schneiden des schlauchförmigen Trennpapiers in Hälften unter Bildung des V-förmigen ersten Trennpapiers (41), das in der Mitte davon gefaltet ist.

2. Verfahren zur Herstellung eines Heftpflasters (1) gemäß Anspruch 1, wobei das zweite Trennpapier (42), das an dem verbleibenden Teil der Oberfläche der wirkstoffhaltigen Matrix (3) haftet, ein folienartiges Trennpapier ist, und das Ende des zweiten Trennpapiers (42), das die Faltung (44) des ersten Trennpapiers (41) bedeckt, zu einer geraden Linie, einer gekrümmten Linie, wie zum Beispiel einer Wellenform oder einer Hügelform, oder einer Kombination davon, geformt ist.

3. Verfahren zur Herstellung eines Heftpflasters (1) gemäß Anspruch 1, wobei das zweite Trennpapier (42), das an den verbleibenden Teil der Oberfläche der wirkstoffhaltigen Matrix (3) haftet, ein schlauchförmiges Trennpapier ist, wobei das schlauchförmige Trennpapier in Hälften geschnitten wird unter Bildung eines gefalteten V-förmigen zweiten Trennpapiers (42) mit einer Faltung, die die Faltung (44) des ersten Trennpapiers bedeckt.

4. Verfahren zur Herstellung eines Heftpflasters (1) gemäß einem beliebigen der Ansprüche 1 bis 3, wobei das erste Trennpapier (41) und/oder das zweite Trennpapier (42) einen Plastikfilm umfasst, gebildet aus Gußpolypropylen, gerecktem Polypropylen, Polyethylenterephthalat, Polybutylenterephthalat, Polyethylen, Polyester, Polyurethan, Polyvinylchlorid oder Polystyrol; Papier, synthetischem Papier, Kunstharz oder einem Verbundfilm, gebildet aus einem Laminat davon; oder einem Verbundfilm, gebildet aus einem Laminat von Aluminiumfolie oder einem Aluminiumabscheidungsfilm.

5. Verfahren zur Herstellung eines Heftpflasters (1) gemäß einem beliebigen der Ansprüche 1 bis 4, wobei das erste Trennpapier (41) und/oder das zweite Trennpapier (42) mit Silikon behandelt wird.

6. Verfahren zur Herstellung eines Heftpflasters (1) gemäß einem beliebigen der Ansprüche 1 bis 5, wobei das erste Trennpapier (41) und/oder das zweite Trennpapier (42) geprägt ist bzw. sind.

## Revendications

1. Procédé de production d'un patch adhésif (1) ayant une structure multicouche comprenant un renfort (2), une matrice adhésive contenant du médicament (3) qui est étalée sensiblement intégralement sur une surface du renfort (2), et une doublure qui adhère à la surface de la matrice contenant le médicament, dans lequel
(a) la doublure adhérant à la surface de la matrice contenant le médicament (3) comprend des première et seconde doublures (41, 42) ;
(b) la première doublure (41) est pliée en son milieu de sorte qu'elle est divisée par le pli (44) en première et seconde sections qui forment ensemble une doublure en forme de V, la première section adhérant à la surface de la matrice contenant le médicament (3) avec le pli (44) agencé plus près du milieu de la matrice contenant le médicament (3) ; et
(c) la seconde doublure (42) adhère à la partie restante de la surface de la matrice contenant le médicament (3) avec une extrémité de la seconde doublure (42) couvrant le pli (44) de la première doublure en forme de V (41),
**caractérisé en ce que** le procédé comprend les étapes consistant à : laisser une doublure tubulaire adhérer à au moins une moitié de la surface de la matrice contenant le médicament (3) étalée sur le renfort (2) ; et découper la doublure tubulaire de moitié pour former la première doublure en forme de V (41) pliée en son milieu.

2. Procédé de production d'un patch adhésif (1) selon la revendication 1, dans lequel la seconde doublure (42) qui adhère à la partie restante de la surface de la matrice contenant le médicament (3) est une doublure de type feuille et l'extrémité de la seconde doublure (42) couvrant le pli (44) de la première doublure (41) est formée comme une ligne droite, une ligne incurvée, telle qu'une forme d'onde ou une forme de montagne, ou l'une de leurs combinaisons.

3. Procédé de production d'un patch adhésif (1) selon la revendication 1, dans lequel la seconde doublure (42) qui adhère à la partie restante de la surface de la matrice contenant le médicament (3) est une doublure tubulaire, la doublure tubulaire étant découpée de moitié pour former une seconde doublure en forme de V pliée (42) ayant un pli couvrant le pli (44) de la première doublure.

4. Procédé de production d'un patch adhésif (1) selon l'une quelconque des revendications 1 à 3, dans lequel la première doublure (41) et/ou la seconde doublure (42) comprend un film plastique formé de poly(propylène) coulé, poly(propylène) orienté, poly(téréphtalate d'éthylène), poly(téréphtalate de butylène), poly(éthylène), poly(ester), poly(uréthane), poly(chlorure de vinyle) ou poly(styrène) ; papier, papier synthétique, résine synthétique ou un film composite formé d'un stratifié de celui-ci ; ou un film composite formé d'un stratifié de feuille d'aluminium ou de film à aluminium déposé.

5. Procédé de production d'un patch adhésif (1) selon l'une quelconque des revendications 1 à 4, dans lequel la première doublure (41) et/ou la seconde doublure (42) sont traitées avec du silicone.

6. Procédé de production d'un patch adhésif (1) selon l'une quelconque des revendications 1 à 5, dans lequel la première doublure (41) et/ou la seconde doublure (42) sont gaufrées.
